# EUROPEAN PATENT APPLICATION

(11) **EP 2 682 885 A2**
(43) Date of publication of application: **08.01.2014**
(21) Application number: 13167196.8
(22) Date of filing: 09.05.2013
(51) Int. Cl.: G06F 19/00

(54) **Tool for deployment of medical services**

(30) Priority: 09.05.2012 GB 201208051
(71) Applicant: Nottingham University Hospitals NHS Trust, Nottingham, Nottinghamshire NG7 2UH (GB)
(72) Inventor: Blakey, John Daniel, Nottingham, Nottinghamshire NG9 3EF (GB); Shaw, Dominick, Nottingham, Nottinghamshire NG8 2FR (GB)
(74) Representative: Adamson Jones

(57) **Abstract**

The invention concerns a medical service deployment system for use in a medical facility. A model of the medical facility is stored on a data storage device. A record of the availability of one or more resources within the medical facility and records of a plurality of tasks to be undertaken within the medical facility are also logged. The records have a task execution parameter and a location of the task to be undertaken in the medical facility. Portable communication devices associated with the resources are in communication with the data storage device, each communication device has location determining means. One or more processors are arranged to compare the availability of the one or more resources with the task execution parameter of one or more tasks to be undertaken and to assign a resource to the one or more tasks in dependence thereon. Tasks may be assigned based on cumulative totals of task execution parameters and/or task locations within the medical facility.

## Description

The present invention relates to the provision of services in a medical establishment, such as, for example, a hospital.

There is a constant need to improve the efficiency with which medical assistance can be provided to patients such that the most critical cases can be handled in a suitable timeframe and with finite resources. In this regard, hospitals have conventionally stood apart from other facilities, such as engineering facilities, in which large numbers of people and associated equipment/tools must be co-ordinated to improve efficiency and avoid adverse situations.

It is conventional practice to record lists of individual tasks that need to be fulfilled within a medical establishment. Those tasks, or a subset thereof, may be assigned to a medical practitioner and recorded with one or more entries, such as the time the task was generated and/or completed. In this manner at least some metrics of performance can be generated. As medical practitioners become available or unavailable (i.e. upon starting or ending shifts) the outstanding tasks may be reassigned by way of a handover, which typically involves a verbal or written description of the circumstances surrounding the patient and/or task to be attended.

In recent years, an increasing amount of data has been collected in secondary health care establishments at the behest of governments, regulators and/or insurance organisations. Such a focus has, at least in part, been enabled by electronic data capture systems in addition to, or in place of, traditional paper records.

However the variety of tasks undertaken in medical establishments and the sheer volumes of data, as well as the different methods of data capture, makes detailed and meaningful analysis of that data difficult to undertake. Thus the potential problems that could be identified and improvements in efficiency achieved are often unresolved and instead the amassed data is used predominantly to report retrospectively on macroscopic indicators of hospital activity. Furthermore, significant factors which affect the efficiency of service at a low level (i.e. at patient and ward levels) may not be captured or recognised at all, and higher level factors (e.g. calendar, weather, special events) may not be considered. The macroscopic, or organisation-level, statistics that are produced have therefore lead to reliance upon a relatively small number of specific large-scale measures of performance, which are summarised, for example, over a period of months or a year.

The inventor believes that the usefulness of those statistics for identifying and resolving performance issues may be improved. There is also significant scope to improve monitoring and deployment systems for medical establishments such that insight can be gained, for example in real time, of the efficiency of the service being provided, thereby allowing resources, such as equipment, beds and materials, as well as personnel, to be deployed or accommodated more effectively.

A more sophisticated system for collecting clinical information and generating performance-related healthcare reports is described in WO 2008/079341 (Marble, et al). However the primary aim of that system is for reporting compliance and assessment of the quality of care given by healthcare providers. Thus, whilst such a system can be used to identify problems in a theoretical context, there is scope for improvement of the implementation of such a system in a way that can benefit the running of a medical establishment at an operational level.

It is an aim of the present invention to provide a tool or system which allows resources within a medical establishment to be tracked and deployed or re-deployed more efficiently.

According to a first aspect of the present invention, there is provided a medical service deployment system for use in a medical facility, the system comprising: a data storage device having stored thereon a model of the medical facility, a record of the availability of one or more resources within the medical facility and records of a plurality of tasks to be undertaken within the medical facility, the records comprising a task execution parameter and a location of the task to be undertaken in the medical facility; a plurality communication devices in communication with the data storage device, said communication devices each being associated with a resource and having location determining means; and one or more processors arranged to compare the availability of the one or more resources with the task execution parameter of one or more tasks to be undertaken and to assign a resource to the one or more tasks in dependence thereon.

The communcation devices may each comprise a portable communication device. The communication devices may each have a display screen. The communication device may be embedded or provided in an equipment resource, such as, for example, as a Radio Frequency Identification (RFID) or other wired or wireless communication means.

The one or more processor is preferably arranged to control the output or transmission of one or more instruction to at least one of the communication devices. The instruction may be indicative of the assignment of the resource to said one or more tasks or vice versa. The processor may assign a plurality of tasks to the, or each, device.

The, or each, communication device may be attributed to, or associated with a resource. The communication device may track the movements and/or location of the resource within the facility. For some resources, which may be static, the location thereof may be stored as part of the model of the facility. Furthermore for some resources of lower value, such as disposable items or devices, the model may comprise the location of a store for such items, rather than tracking the location of individual items.

The one or more processors may also compare the location of the task to be undertaken with a location of the one or more resources and assign a resource to the task based, at least in part, upon the distance there-between.

The record of the availability of one or more resources within the medical facility may comprise an indication of whether the resource is present within the facility. The record of the availability of one or more resources may comprise a location of the resource, for example with reference to the model of the facility.

The record of the availability of one or more resources may comprise an indication or record of the tasks currently assigned to the resource. The record of availability may comprise a total of the task execution parameters for the tasks currently assigned thereto.

The record of availability for each resource may have a threshold value. The threshold record of availability may be a predetermined or preset value which may be specific to that resource. The processing or comparing of the records may comprise determining a record of availability for a resource, for which the current total of task execution parameters is below the threshold value.

The processing or comparing of the records may comprise determining a record of availability for a resource, for which the magnitude of the difference between the current total of task execution parameters and the threshold value is greatest (e.g. the lowest total, relative to the threshold value for that resource). This may comprise comparing the task execution parmeters for a pluarilty of resources and selecting a resource from said plurality based on said magnitude determination.

The resource may be a human resource, medicament and/or equipment resource. The equipment resources may comprise any, or any combination, of medical machines (including beds or wheelchairs), medical devices, rooms (such as operating theatres or other rooms including the equipment therein) and/or consumable materials.

In the embodiment of human resources, the availability of the resources may comprise a record of the personnel currently at the facility. The record may comprise a record of the duration for which each member of staff will be available for example by way of a record of the time at which each member of staff is due to leave the facility. That is to say the record typically accommodates personnel shift patterns and/or schedules.

A plurality of roles or profiles may be defined for the resources. Each role or profile may be indicative of a different type or level of resource. Each role/profile may have associated therewith a list of tasks or types of task that can be undertaken by a resource. For either a human or equipment resource, first and second profiles may be defined. For example each resource may have more than one profile assigned thereto, which is typically stored as an entry against the resource within the data store. A first profile may be indicative of a type or hierarchical level of the resource and may comprise a general or coarse profile. A second profile may be indicative of specific tasks which have previously been, or can be, undertaken by the resource. The tasks indicated by the second profile may be a subset of, or additional to, those listed in the first profile.

The task execution parameter may be indicative of the severity or urgency of the task. The task execution parameter may comprise a score or weighting. The task execution parameter may additionally or alternatively comprise a predicted or estimated duration of the task to be undertaken. The task execution parameter may additionally or alternatively comprise a predicted amount or number of resources for the task. In any embodiment, the task execution parameter may be recorded as a numeral. A plurality of task execution parameters may be recorded for any or all tasks.

The records of the plurality of tasks to be undertaken may comprise any or any combination of: a time of input or creation of the task record; a time of assignment of a resource to the task; a status of the task; a time of status change of the task; a time of reassignment of the task; and, a time of completion of the task. In any embodiment, the status of the task may have an attribute indicating whether the task is assigned or unassigned (or, possibly, re-assigned). The one or more processors may thus assign unassigned tasks in order of entry or according to their task execution parameter.

The task records may additionally or alternatively comprise a record of task type. A record of the applicability or suitability of different resources for each of said types of task may be maintained.

The model may comprise a plurality of portions. Each portion may be distinguished from a further portion by any or any combination of physical location, function and/or organisational structure. The model may comprise a departmental model, for example comprising a plurality of wards or similar delineations.

The model may comprise a graphical model or plan for the medical facility, such as a floor plan, or plurality thereof. The location of the task to be undertaken may correspond to a location or portion of the model. A marker for the task may be provided at the task location in a graphical display of said model. The task location marker may comprise a graphical indication of the associated task execution parameter. One or more resource location marker may be provided in the model.

A cumulative total or summation of the task execution parameters for a plurality of active tasks may be maintained. Such a total may be provided for one or more portions of the facility or else for the facility as a whole. Such a total of all active tasks in said portion(s) or said facility may be maintained. The one or more processors may compare the total for one portion of the facility with the total for one or more further portions of the facility. Tasks and/or resources may be assigned accordingly, for example so as to balance the totals between different portions of the facility.

The availability of a resource may be determined based upon a summation of the task execution parameters of the tasks assigned to that resource

The cumulative total may advantageously provide a single, universal attribute which can be used to compare different facilities or parts thereof or individual resources. Such an attribute may be considered to provide a score or else an indicator of the level of activity in the facility or a portion thereof. Having such a universal, tradeable attribute is particularly beneficial since different parts of a facility, such as different departments, wards or functions, can be compared against other parts. If the resources available to one part are overstretched, then the resources of another part of the facility can be assigned to help share the tasks/workload in a managed and efficient manner.

In one embodiment the one or more processors may determine a risk indicator for the facility or a portion thereof based on both the cumulative total (e.g. the score) and the record of the availability of said resources. The record of the availability of said resources may also comprise a cumulative value, for example of all the resources, or a type thereof, available in the facility or the relevant portion. The risk indicator may be determined based on a ratio of the score and record of availability, or vice versa.

As with the total activity score, or else instead of that score, the risk indicator provides a universal, tradeable attribute which can be used to compare facilities or portions thereof in real time.

In one embodiment, a threshold value of risk or total activity or score may be set for one or more resources, one or more portions of the facility or else for the facility as a whole. Such threshold values may be predetermined, for example based on experience of adverse event criteria, or else may be determined and/or amended using the tool of the present invention. The one or more processors may determine the ability, or availability, of a resource or portion of the facility to undertake further tasks based on whether or not the threshold value has been met or exceeded.

The one or more processors may generate an alert and/or reassign one or more task to a different resource if a threshold value is met or exceeded. The one or more processors may assign tasks and/or resources between different portions of the facility in the event that the threshold is met or exceeded.

The data storage device preferably comprises a central data store, which may be accessible by a plurality of computing devices. Said computing devices may comprise the one or more processors. The computing devices may comprise the portable communication devices and/or a static workstation. The system may comprise a communications network, such as a local wired and/or wireless communications network, which may comprise a secure network. Any, or any combination, of the data storage device, the portable devices and/or the one or more processors may comprise the relevant transmitter and/or receiver hardware.

The position determining means may comprise a GPS receiver, typically having associated circuitry for position determination based on received satellite position signals. Additional or alternative signal transmission/reception protocols/standards may be used to determine location. For example Wi-Fi (RTM) transceiver locations may be used to determine location of a resource within the facility. In one example a plurality of Wi-Fi transceivers/routers within the facility may be used to determine the location of one or more resource.

In one example, the position determining means comprises movement detection sensors, which may be used instead of, or in addition to, wireless communication signals. An inertia sensor may be used to this end, which may be coupled to the resource itself and/or the portable communication device. The inertia sensor may be worn by a user. Inertia sensor data may be used in conjunction with GPS and/or Wi-Fi signals to more accurately pinpoint the location of resources with respect to the model of the facility. This is particularly useful indoors to pinpoint the location of resources relative to internal corridors, rooms, walls or any other structure of the building.

The data pertaining to completed tasks, or a subset thereof, may be stored for subsequent interrogation and/or for statistical analysis of the tasks undertaken over a period of time.

Each task may have a task identifier indicative of the type of task to be undertaken. A predetermined list of task identifiers may be maintained, for example on the data storage device, or accessible thereto. The task identifier may have associated therewith one or more further tasks. The task identifier may comprise an attribute linking it to one or more further tasks which are dependent thereon or therewith, for example so as to define a time-ordered or hierarchical sequence of tasks. Such linking attributes may be hierarchical. For example, an ordered sequence of tasks may be defined thereby, such that a later task may be dependent upon the completion of a prior task. One or more later task may be assigned provisionally (e.g. before the prior task is completed), for example at the time of assigning the prior task. Alternatively, a later dependent task may be assigned only upon completion of the prior task.

The maintenance of a predetermined list (e.g. a white list) of tasks helps to ensure that tasks can be assigned reliably and effectively to a suitable resource. Thus task assignment can be, at least in part, automated.

Any, or any combination, of the data storage device, the portable/communication devices and/or the one or more processors may have a free-text entry function. The one or more processors may analyse the entered text and may categorise or interpret the meaning, subject or nature thereof. A log of the results of said analysis may be maintained at the data store. Text entered using the free text entry function may be logged. Accordingly the system may comprise a text parser. Records of the count of tree text entries pertaining to a particular topic, such as a task, type of task, a resource or the availability thereof, may be maintained. Reports and/or alerts may be generated based upon such records or the count thereof.

Whilst the limited number of predetermined tasks allows the system to operate efficiently and effectively, the provision of a free-text entry field, or so-called "grumble" function allows the system to accommodate non-standard situations and their impact on the system operation. This function has been found to be particularly useful to the successful implementation of the invention.

Any, or any combination, of the data storage device, the portable devices and/or the one or more processors may have a visual or graphical display associated therewith. A visual representation of the facility model may be output on the display. The location of tasks, such as active tasks, may be indicated on the visual representation. Tasks may be represented by visual or graphical indicia on the visual representation. Tasks may be provided with an indicator of urgency, for example by colour coding or labelling of the task indicia.

Additionally or alternatively, the visual display may be used to present details of one or more tasks assigned to a resource, such as, for example, a user associated with that device. A list of tasks to be undertaken may be output on the visual display.

In any of the above described embodiments, the one or more processors may comprise one or more routines or algorithms for automatically implementing any of the steps described above. A manual over-ride function may be provided such that an operator can manually assign tasks and/or resources as necessary.

According to a second aspect of the invention, there is provided a method of operating, or deploying services in, a medical facility comprising: entering onto a network-accessible storage device records of a plurality of tasks to be undertaken within the medical facility, the records comprising a location of the task to be undertaken in the medical facility; maintaining a model of the medical facility and records of the current availability of a plurality resources within the medical facility; assigning a task execution parameter to each record of a task to be undertaken; processing the records of the current availability of said resources based upon the task execution parameters and assigning a task to the one or more resources in dependence thereon.

According to a third aspect of the invention, there is provided a data carrier comprising computer readable instructions for the operation of one or more processors to receive and/or process records of a plurality of tasks to be undertaken within a medical facility, the records comprising a location of the task to be undertaken in the medical facility; access a model of the medical facility and records of the current availability of a plurality resources within the medical facility; receive or assign a task execution parameter to each record of a task to be undertaken; process the records of the current availability of said resources based upon the task execution parameters and assign a task to the one or more resource in dependence thereon.

According to a fourth aspect of the invention, there is provided a medical service deployment tool for use in a medical facility, the tool comprising: a data storage device having stored thereon a record of the availability of a plurality of resources within the medical facility and a record of a plurality of tasks to be undertaken within the medical facility, the task records comprising a task execution parameter and the record of availability for each resource comprising a total of the task execution parameters for the tasks currently assigned thereto; and one or more processors arranged to compare the availability of the one or more resources with the task execution parameter of one or more tasks to be undertaken and to assign a resource to the one or more tasks in dependence thereon.

The data storage device may have stored thereon a model of the medical facility. A record of the availability of a resource may comprise a location of the resource within the facility. The tool typically comprises a display screen for displaying alerts and/or reports generated by the one or more processors.

According to a fifth aspect, there is provided a method of provision of medical services corresponding to the fourth aspect.

According to a sixth aspect of the invention, there is provided a model of a medical facility, the model comprising: records of the availability of a plurality of resources within the medical facility and records of a plurality of tasks to be undertaken within the medical facility, the records comprising a task execution parameter and a location of each task to be undertaken; wherein an output of said model comprises a graphical display of the facility including the location of the tasks and/or resources such that the distance between tasks and resources is determinable.

In the above described aspects or embodiments thereof, the or each record may comprise a current or "real time" record. Additionally or alternatively, the or each parameter may comprise a current or "real time" parameter value. Thus the system or method of the invention may be updated in real time so as to adapt to the changing events and status within the facility. The system and/or method is also scalable such that it can accommodate tens, hundreds or thousands of concurrent tasks as well as the associated records, which may be active within a medical facility.

In any of the above-described aspects or embodiments thereof, the facility may otherwise be referred to as an establishment. The facility may comprise one or a series of buildings, which may for example be located on a common site or within a common vicinity. In one embodiment, the system and/or method of the present invention may advantageously allow co-ordination of medical services over a geographical area or locality, such as between a plurality of facilities, for example within a town, city or region.

Any of the optional features described above in relation to any one aspect of the invention may be applied to any other aspect wherever practicable.

Working embodiments of the present invention are described in further detail below by way of example only with reference to the accompanying drawings of which:
Figure 1 shows a schematic of the hardware components of a system according to an example of the invention;
Figure 2 shows a schematic of the information and data flowing through a system according to an example of the invention;
Figure 3 is an example of a user interface of one embodiment of the invention showing a floor plan of a medical establishment;
Figure 4 shows the procedural steps undertaken in an example of use of the invention;
Figure 5 shows a display generated by one example of the invention, indicating a route to the location at which a task is to be undertaken;
Figure 6 shows a display generated by one example of the invention, showing activity indicators for a medical facility;
Figure 7 shows a display generated by one example of the invention for the purpose of reporting past activity; and
Figure 8 shows a display generated by one example of the invention for the purpose of reporting resource movement within the facility.

The invention may be considered to provide a system for extracting, combining, displaying, and analyzing healthcare-related measures to provide a measure of current performance of a healthcare facility, or portion thereof, thereby allowing the deployment of medical services to react to transient conditions. The system provides location modelling and tracking functionality to allow more efficient deployment of resources. The system may additionally or alternatively provide an early warning of potential adverse outcomes. The system can also respond to early warning triggers and/or location information to adjust other systems, deploy equipment, or notify personnel.

The invention may be considered to provide a "Secondary care Performance Assessment and Response Kit", for which the inventor has coined the name SPARK^{™}. Whilst the invention is intended to be bespoke to medical facilities, it is appreciated that other possible uses for the invention may be found in different types of facilities. However in general medical facilities typically provide a unique set of problems, in particular due to the criticality of the services provided.

The invention provides a tool for networked hardware that combines augmented data from hospital sources in order to provide real-time data/information on measures and locations of activity. Such real-time monitors can be generated and output for a plurality of different levels of organisation for a medical facility, or a plurality of medical facilities, such as, for example at any of individual, ward, department, whole facility, or other bespoke levels, including regional or directorate levels, which may accommodate the performance of a plurality of medical facilities over a designated geographical area. The scalability of the invention is thus one important advantage over the prior art.

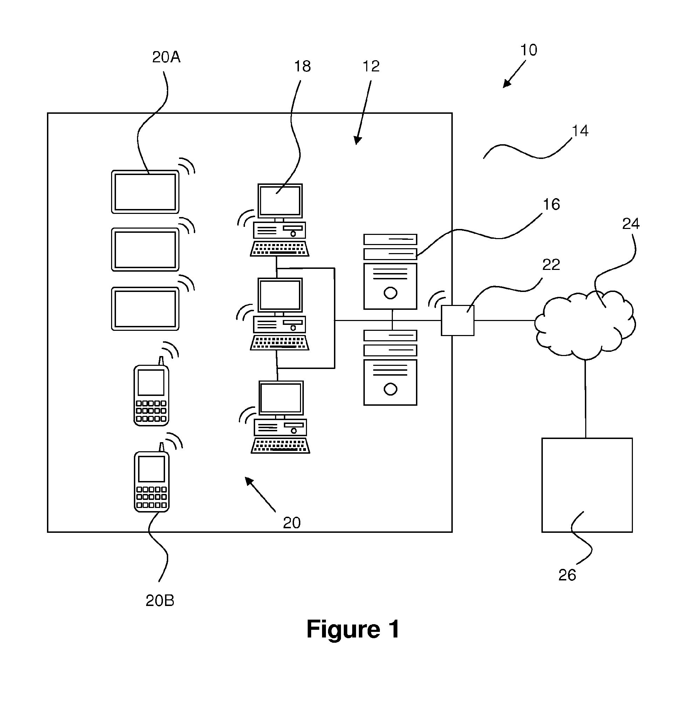

Turning now to Figure 1, there is shown a schematic example of a hardware system or configuration to which the invention can be applied. The system 10 comprises a local area network 12 for a medical facility, in the form of a hospital 14. As will be appreciated by the person skilled in the art, the medical facility may comprise a single building or a plurality of buildings/premises spaced over one or more sites. The network may thus accommodate a local and/or wide area network as necessary. In any embodiment the network will typically comprise a secure network, having the necessary firewalls, thereby preventing unauthorised access to the network-accessible resources.

The network 12 comprises one or more common data stores 16 which may be accessed by a plurality of devices on the network. The one or more data stores may thus provide a master copy or version of the data which is accessible over the network in accordance with the invention.

In various embodiments of the invention, the devices may access data from the data store by way of a wired and/or wireless connection. In this embodiment, the devices comprise a plurality of wired devices 18, which typically take the form of personal computers or other types of workstation, and a plurality of wireless, typically portable, devices 20. The wireless devices are preferably wireless communication devices and may take the form of any or any combination of tablet computers 20A, mobile telephones 20B, laptop or notebook computers, PDA's or the like. Each device has associated therewith a display screen and one or more user input controls, such as a keyboard, mouse, keypad or other button configuration, or else a touch screen interface.

The portable devices 20, each have position determining means in the form of a signal receiver and associated circuitry for determining the location of the device from the received signals, for example from satellites. The portable devices may have a GPS receiver for this purpose. Additionally or alternatively, the portable devices may make use of other techniques for location determination, for example by reference to the signal strength of one or more received radio signals, for example from a plurality of Wi-Fi (RTM) access points or else from one or more telecommunications antenna masts or base stations. Software may be installed on the device for the purpose of analysing the incoming signals and determining the location of the device.

However it has been found that the specific problems associated with location determination within buildings (e.g. signal degradation though the building structure) as well as the accuracy of determination required for the present invention can lead to conventional GPS systems being insufficient. Accordingly it has been found that the use of a bespoke movement sensor, such as an inertia sensor worn by a user or located on the resource or communication device, can significantly improve accuracy. Wireless communication signals may thus be used to provide a macroscopic location indication for a resource and the inertia sensor may be used in conjunction therewith to provide an account of the movement of the resource through the facility so as to pinpoint the resource location.

Network communications are handled by one or more conventional server and/or router devices 22, which may comprise conventional wired/wireless hardware as would be understood by the person skilled in the art. A server 22 may provide a connection to the internet 24, thereby allowing for connection, typically in a secure manner, to another facility 26, which may have a network as described above in relation to the network 12, or the different embodiments thereof. In the case of multiple facilities or networks, a common data store for all the facilities may be provided or else each facility may have a dedicated data store which may receive data from one or more other facility.

Within the network 12 a number of levels of access may be defined, including, for example, an administrator level and an end user level. The higher level of access allows for creation and/or deletion of tasks as well as permissions to enter/amend a wider variety of data types and access to reporting tools as will be described below. An end user level of access may allow restricted access, for example, only to update certain data fields for tasks assigned thereto.

Machine readable instructions in the form of one or more software packages are loaded onto the devices 18, 20 within the facility 14. The software is used to control the access to, including the writing/reading of data to/from, the data store 16. Thus the software generates one or more user interfaces providing customised user input fields, controls and reporting tools and the software interprets the user inputs, such as data and/or control inputs, in order to provide the necessary output to any, or any combination of, the user, another device and/or the data store 16.

Data will typically be encrypted for storage and transmission using conventional medical-grade encryption.

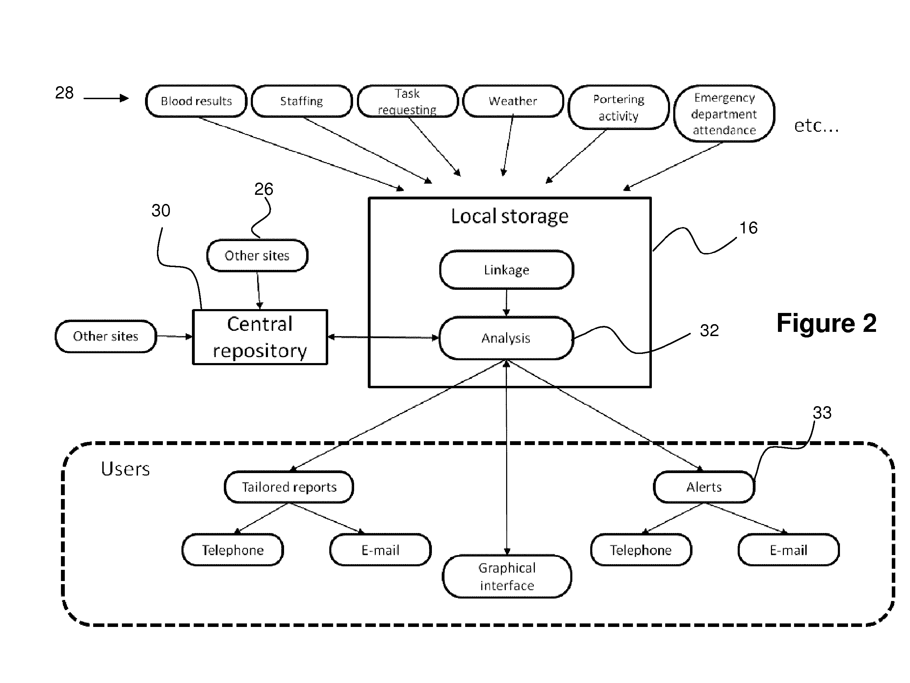

Turning to Figure 2, the software and associated user interfaces allow data to be captured from multiple input sources 28. One example comprises data entered by a user, typically an administrator, upon admission of a patient to the hospital or a ward thereof. Upon admission a patient, the user will typically enter admissions data in a conventional manner. However in accordance with the embodiments of the invention, the user will also be able to identify and select one or more tasks to be undertaken for that patient, such as, for example: the taking of samples (e.g. a blood or urine sample); the measurement and/or recording of a patient's temperature, blood pressure, etc; performing a scan or other medical imaging (e.g. X-ray, MRI, ultrasound); portering; or other designation of equipment (e.g. a bed), task or human resources for that patient.

The user will also be able to access/see via the user interface any tasks that have been requested or entered by other users for that patient. Thus a patient record comprising all tasks entered for that patient is made available.

The time of creation of each task can thus be recorded along with its status and a type or kind attribute for the task, by which the software can recognise the task from a predetermined list of tasks. The predetermined list of tasks comprises a number of tasks which have been previously entered as having a set of attributes. Those attributes may comprise a record of: the equipment required for and/or to be consumed by a task; and/or the type or level of medical practitioner that is able to perform the task. In this regard each resource has assigned thereto a basic profile indicating a type of resource and the tasks attributed to that type. For personnel, this basic profile may indicate the level, grade or nature of their role. A more customised profile is also provided which contains bespoke tasks suitable for that particular resource. For personnel, this customised profile comprises a personal profile which is maintained by the individual, and in which the individual can select specific or deselect tasks from a predetermined list, based on their individual competencies and/or experience. A parallel system applies to equipment resources, wherein different models or instances of equipment of a common type can be identified as offering different functionality (e.g. different scanners having different functions, or else different operating theatres having different equipment therein)

Additionally or alternatively, the task type attribute comprises a task execution parameter in the form of a score, weighting or rank, such as an alphanumeric symbol or string, which provide an indication of the level or resource required by the task. This parameter may therefore provide an indication of the urgency, severity, criticality and/or intended duration of a task. Such a parameter is particularly important in allowing comparison and assessment of tasks in a hospital environment wherein tasks can vary significantly from patient to patient and from ward to ward.

The task execution parameter may additionally or alternatively comprise a location parameter. The location parameter may comprise an alphanumeric symbol or string, which identifies a physical location within the facility (typically with respect to the facility model), at which the task is to be performed.

According to aspects of the invention, the task identifier may comprise an attribute linking it to one or more further tasks which are dependent thereon or therewith, examples of which will be described below. This linking of an initial task to one or more downstream tasks which will result or be required upon execution of the initial task is an important feature since it allows later tasks, and the resources there-for, to be predicted in advance. Accordingly the invention can predict downstream stresses on resources that will naturally result from current circumstances, allowing a user to plan accordingly.

Each task also has an assigned/unassigned attribute to determine whether or not all the resources required for completion of the task (personnel and/or equipment) have been assigned to the task. Each task type will thus typically also have a resource parameter or field to indicate what resources have been assigned to the task. The time of assignment/reassignment of a task is also logged.

It is to be noted that, in the case of standard consumables, there may be no need to specifically apply the required equipment and it can be assumed that such consumables are generally available to medical personnel. However the logging and completion of tasks can still be used to keep a count of all equipment (including consumables) usage as will be described below.

The location of more important/valuable equipment (i.e. higher valuable and/or reusable assets) and/or the storage location of consumables within the facility is recorded as part of a facility model to be described below.

For completeness it is noted also that medicaments, such as pharmaceutical products, may be considered to be an example of "equipment" as used herein, although in a typical embodiment of the invention, medicinal drugs and equipment will be listed as separate resource types.

Whilst the above example of patient admission will typically be a common scenario in which tasks are created, it is to be noted that tasks can be generated at a plurality of different locations or stages of care for a patient.

The data inputs/sources 28 shown in Figure 2 may include any or any combination of the following, which are provided purely as a non-exhaustive list of examples: blood result requests and results; radiology requests and results; emergency medicine department activity; admission ward activity; staffing levels (number, type, seniority, and location); junior doctor on-call activity; portering activity; nursing activity; the location and distance travelled of selected staff; the volume/number, nature, and duration for out of hours tasks; adverse event and incident data; cardiac arrest calls; trauma calls; theatre use; emergency theatre waiting lists; date; weather information; equipment use and failure (e.g. CT scanner, haematology laboratory analyzer, etc); length of stay statistics; ward closures; number of empty beds; consumable ordering and stocking; equipment library use; equipment service logs; and, electronic drug prescribing records.

Data from other sources such as staff satisfaction surveys, patient complaints, and financial performance can also be captured as comparators for these input data.

The input data may also include information entries made by users via a feature of the system herein referred to as "the grumble button". Such a feature may otherwise be referred to as a minor incident or "near miss" free text reporting function. This is an intuitive system whereby the user can enter text into a suitable free text entry interface (e.g. a text box) on their device 18, 20. Each text entry may be limited to a maximum number of characters, such as 140 in this example. In this box the user can confidentially record minor incidents (or situations that had the potential for incidents to occur) that they feel do not warrant the investment of time required in recording in a lengthy and complex formal incident form or that need a more rapid response. These grumbles could comprise a failure of equipment, a delay in obtaining or contacting a resource, or being unable to locate equipment in the designated location. The grumble text entry function is activated by actuation of a dedicated a key on their device, which may comprise a physical or on-screen key.

The system comprises text parsing software, typically loaded on a central processing device or station, receives the text messages sent by the users via the grumble button. This system conducts text sentiment analysis to detect key words and phrases as being indicative of a particular meaning. Conventional tools of this kind maybe employed to categorise the meaning of messages based on the words used and their context and/or phraseology. Certain predetermined meanings may trigger an immediate response, e.g. if a user made negative comments about life-critical equipment. Such responses may include the assignment of a task to a further suitable user, such as a manager or other senior-ranking medical professional, to verify the situation or veracity of the grumble.

Other grumbles of lower priority may not require immediate response. However all entries made using the grumble function will be tallied, once the intent thereof has been determined, in order to keep a count of the different types of grumbles received and from where. This free text entry, "grumble", function is considered an important implementing feature for embodiments of the invention in that it allows account to be taken of factors which fall outside of the parameters formally recorded by the service deployment tool. Accordingly this function can be used to address the under-reporting of incidents in healthcare and would keep management abreast of a variety of potential issues or concerns.

All the collected data is stored in a suitable format within the local data store 16, such that it is available for analysis and decision making substantially in real-time and also for later reporting purposes. By "real-time" it is intended that data is made available for processing and/or analysis within the time frame for completion of an active task.

A key feature according to aspects of the invention is that all data is stored and linked by source identifiers (e.g. patient identifier number, ward location, junior doctor responding, equipment used). This allows, for example, multiple issues with one piece of equipment to be handled differently by the system to one issue with each of many machines, or for example multiple abnormal blood biochemistry results to contribute less to the risk score if arising from a renal ward than a respiratory ward. The system also allows recorded data to be manually edited (with a record of the user making changes) if inaccuracies are introduced by human error (e.g. mislabeled blood tubes, wrong ward code).

Also shown in Figure 2 is the framework for provision of a central data repository 30 for access and co-ordination between multiple medical facilities or sites.

The analysis stage 32 shown in Figure 2 in accordance with the various aspects of the invention comprises the processing of the amassed task data to determine the relative burden or strain exerted on individual resources or between different portions (e.g. wards or departments) of the hospital or else between different facilities or portions thereof. For example, using the structure shown in Figures 1 and 2, corresponding wards or departments in the same or different facilities can be compared within a common framework. This allows determination of inefficiencies or bottlenecks, which can be resolved, e.g. at lower levels of organisation, in real-time or else which can be logged and reported to allow efficiency-improving changes to be planned and/or implemented in a longer timeframe.

The analysis stage may be performed on dedicated hardware, which is associated with the data store, or else more typically can be performed by the devices 18, 20 used to access the data store. It is envisaged that a majority of routine processing can be performed by devices 18, 20, whereas high-end processing, involving larger volumes of data (e.g. over longer time periods) and/or more complex routines may be run on dedicated hardware for more detailed reporting.

A core function of the analysis stage 32 is to maintain a real-time assessment of the data execution parameters for the different portions/functions within the facility. For the task scores/weightings described above, this is achieved by maintaining a total of the active

(i.e. created or assigned tasks, which have not yet been completed) task execution parameters for the different portions of the facility. In the example of high value equipment or assets, the task execution parameters assigned to that resource can also be determined for comparison with other equipment of a similar type.

In this example, the task execution parameter is provided in the form of a numeric score for each task, which may be considered to provide a weighting or risk index value. A predetermined scale is provided for all tasks, such as from 0 to 10, 20, 50 or 100, dependent on the level of granularity required by the medical facility. Each task is provided with a score on this scale, which may for example be a predetermined characteristic of the task within the stored task attributes. Accordingly each task of the same type will typically have the same score, at least initially.

In one example, this score may then be updated over the time for which the task remains uncompleted, for example in an automatic manner (e.g. on a sliding scale) or else manually in response to changing conditions. Thus, for example, the need to collect a sample from a patient could be a low-priority task or high-priority task, with an associated score, depending on the condition of the patient.

This score attribute allows meaningful comparison of different types and categories of tasks in dependence on the type of task and/or its urgency/age. A user inputting or updating a task may be prompted via the user interface to confirm the currently assigned task score prior to exiting the task record.

The total activity for a resource, ward, department, facility or number of facilities can be maintained by the summation of all active tasks assigned thereto or else pending assignment thereto. This scoring system is particularly beneficial in that a common logic is scalable from an individual level all the way up to a whole-organisational level.

At the analysis stage 32, as well as determining the total activity score for one or more resources, one or more algorithms can be run to determine further conditions and/or respond thereto.

In one embodiment, the total activity score for one or more resources is compared to a threshold value, for example to determine whether the resource can cope with the tasks assigned thereto. Threshold values may be predetermined for individual resources, portions of the facility and/or the facility as a whole. If a threshold value is met or exceeded, the system will typically take one or more responsive actions. A most fundamental action comprises the outputting of an alert 33 to the relevant device or user, for example by way of an email, text message, push notification to the relevant device 18, 20. Additionally or alternatively, a report may be output, typically to a user's display screen to provide a more detailed account of the level and/or type of activity. Such reporting tools are discussed in more detail below.

In addition to the total activity score, a risk rating or score may be determined based on the ratio or percentage of the current total activity score to the threshold value. A number of other factors may also be taken into account in the determination of a total risk score. Such combined factors may be weighted in order to balance their affect on the total risk. Responsive action may be taken if the ratio or percentage exceeds a predetermine threshold for that value, such as at for example 70, 80 or 90% of the predetermined maximum capacity. This is useful in that an early warning can be provided and responsive measures to resolve impending issues can be taken before critical levels of activity are reached. Alerts and the like thus allow manual intervention at an early stage.

In a further embodiment, if any of the thresholds described above are met or exceeded, the one or more processors may re-assign one or more tasks so as to reduce the total activity score for that resource or portion of the facility. In the event that multiple facilities use the system, tasks may be reassigned between facilities (for example between corresponding departments or resources in those different facilities). Tasks/resources may be reassigned with the aim of reducing the total activity score to below the threshold value. This may be done (e.g. automatically) by searching for a suitable resource with the lowest total activity score, or else for which the total activity score is sufficiently below its threshold value. In more detailed embodiments, an iterative scheme may be employed in which the higher levels of activity score (i.e. for apportion of the facility or the facility as a whole) are acceptable prior to reassignment.

In any embodiment, the threshold levels are values that have been previously recorded or experienced as being associated with adverse outcomes such as, for example, drug administration errors, cardiac arrests, delayed discharge, or avoidable high costs. The tool therefore acts not only as a measure of activity and performance but also as an early warning system that can highlight potential problems before they occur, and take steps to begin to address risks. This aspect of the SPARK^{™} tool facilitates the deployment of a responsive system where additional staff with specific skills can be automatically alerted or moved, or additional equipment deployed if the risk of error or adverse outcome is high.

In one embodiment, the initial assignment of a task may be automated in the same manner, for example by determining which of the suitable resources has the lowest current total activity score relative to its threshold value. In any such embodiments, the assignment or reassignment may be automated or semi-automated, for example by providing a suggestion to an operator, such as an administrator, prior to task assignment. For smaller numbers of resources, (e.g. at an individual ward level), an operator may manually check the total activity scores of available resources and decide how best to assign a task.

In automated or semi-automated embodiments, pre-specified action is taken in response to the exceedence of a threshold. Options for such response can be established during set-up of the tool so local factors can be taken into consideration.

If a concern threshold is reached (or action is triggered by "grumble button" comments), the system can perform a number of functions in response, of which examples include alerting individuals already working to take a specific action (e.g. moving equipment or consumables to a specified location, checking the functioning of a piece of equipment, cancelling an operating list, cleaning a contaminated area); alerting individuals not at work to take action (e.g. a surgeon required for a specific operation, a maintenance engineer for a failed dialysis machine, a manager to open an overspill ward); and interacting with other hospital systems (e.g. to order more blood taking equipment, to keep a scanner running as a further scans are likely to be needed). E-mail or text alerts can also be generated for external agencies (e.g. to alert the ambulance service that their crews may face long waits to off-load patients, or to manufacturers of failing equipment).

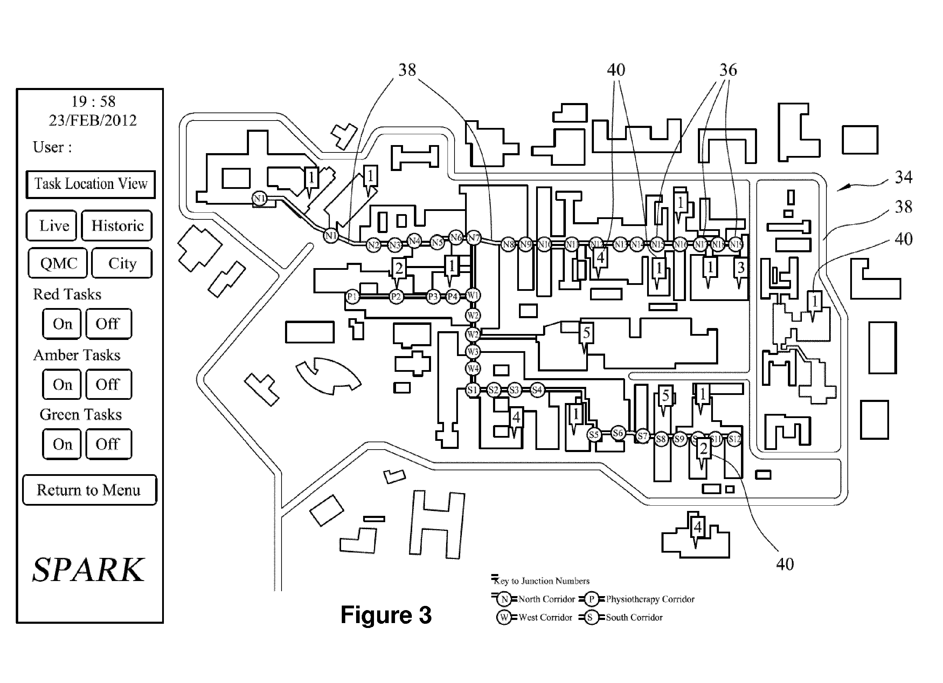

Turning now to Figure 3, there is shown an example of a facility model which is maintained by the data store 16 and/or devices 18, 20. The facility model comprises a spatial model or plan 34, a representation of which can be output on a visual display screen or as a printout. The model differs from a simple schematic in that the actual distances between locations in the model is accurately modelled. Furthermore walls or other portions of the medical facility may be modelled as being impassable such that the model contains an accurate representation of the accessible areas of the facility and the routes by which they can be accessed.

This mapping is achieved by recording the relative location of a plurality of points 36 and routes 38 in the model. The points may be considered to be nodes and may comprise the location of for example: wards, departments; secure entrances, exits, which may be external or internal (e.g. to wards or areas within the facility); the location of resources such as equipment or stores thereof; and/or junctions within the routes of the model. The routes typically comprise walkways, such as corridors, or, for larger facilities, may also comprise vehicular routes such as roads or paths/tracks. In this manner the distances between locations in the facility can be accurately determined via the available routes.

The active tasks 40 are displayed/output on the model representation 34. In this example, each task is provided with visual indicia such as a marker or icon at the location of the task. In this particular example a numeral is provided within, or otherwise associated with, each marker 40 to indicate the number of tasks to be undertaken at that location. Additionally or alternatively each marker 40 or location 36 is provided with a visual indication of the level of activity at that location, for example by colour coding of the marker/location. A traffic light system may be used to indicate the level of activity. In alternative embodiments, a numeral, such as the summation of the task execution scores could be used for this purpose.

This system has been found to provide a particularly useful planning and reporting tool in contrast to purely textual outputs. The graphical output may take the form of a GUI as shown in Figure 3, which may comprise one or more user interaction keys or buttons to allow the display to be modified as required, for example to show and/or hide task details or other information on the plan 34.

As well as the known location of tasks/points within the model, the location of the devices 18, 20 is also known. For fixed devices, the location may be fixed permanently or semi-permanently such that it does not need to be tracked. However for portable devices 20, a log of the current location of the device is maintained and is accessible to the one or more processors performing the analysis stages 32. In this regard, the position determining means of the portable devices records of the location of the devices, which location record may be logged at suitable time increments in order to track movements of the device owner/operator. The location may be recorded with respect to the facility model.

In some embodiments, it has been found beneficial to provide fixed reference points of known location within the facility such that location with reference to those fixed locations can be accurately determined. This is particularly useful if using a movement sensor of the type described above such that movement/inertia readings for the sensor can be used to determine position relative to one or more fixed starting point. Such sensors can thus determine distance travelled.

In some embodiments, analysis stage 32 may take into account the distance to travel between a resource and a task. Tasks may thus be assigned, at least in part, based on distance between the resource and task to be completed. For example, if a number of possible resources are identified as being suitable to handle the task, a particular resource may be selected based on proximity to the task or patient location. Additionally or alternatively, for high priority tasks, the selection of the closest resource may override any other factors taken into account by the analysis function during normal operation.

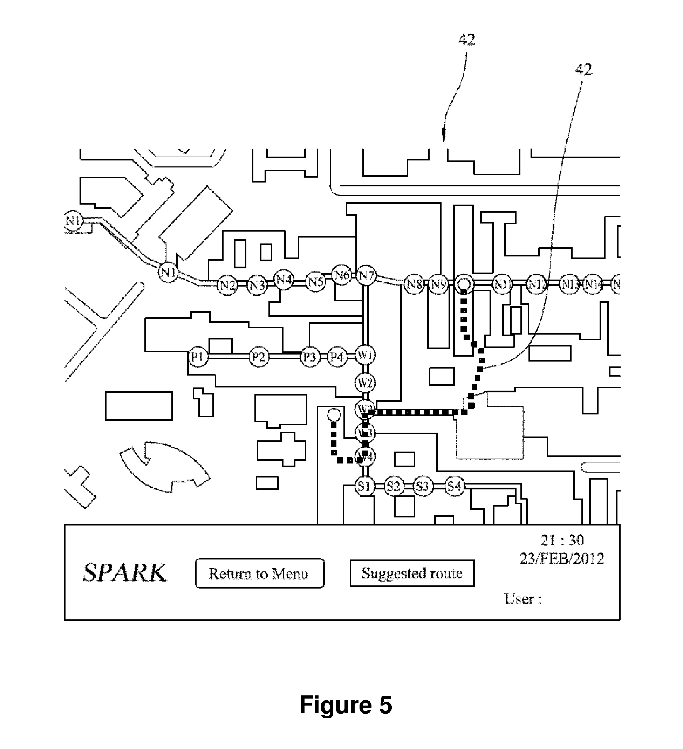

For any type of task, once assigned to a resource, the analysis function 32 can determine any possible routes from the resource's current location to the task (or vice-versa) and the distance thereof. A shortest route can thus be selected and displayed to a user on a screen of their device 18 or 20. An example of such an output is shown as the graphical user interface of Figure 5 which shows the relevant portion 42 of the facility model 34 with the suggested route 44 overlaid thereon. This can be used to guide a user to the location of the task or equipment resources (e.g. a request to undertake a chest drain could include the fastest route to the ward via the nearest location of portable ultrasound machine and chest drain equipment). The display can also to be used to help explain to a patient where they need to be in the event that they need to move to another part of the facility for the task at hand.

For portable communications devices, a log of distance travelled will also typically be maintained to determine any losses in efficiency incurred due to resources in transit. This may help establish where existing or planned resources may be better deployed in the facility. Such a route planning and/or logging function is of particular benefit to those who are new to the hospital, e.g. locum staff. It may also be updated with changes to the facility, such as maintenance works, corridor closures, new facilities, equipment and the like.

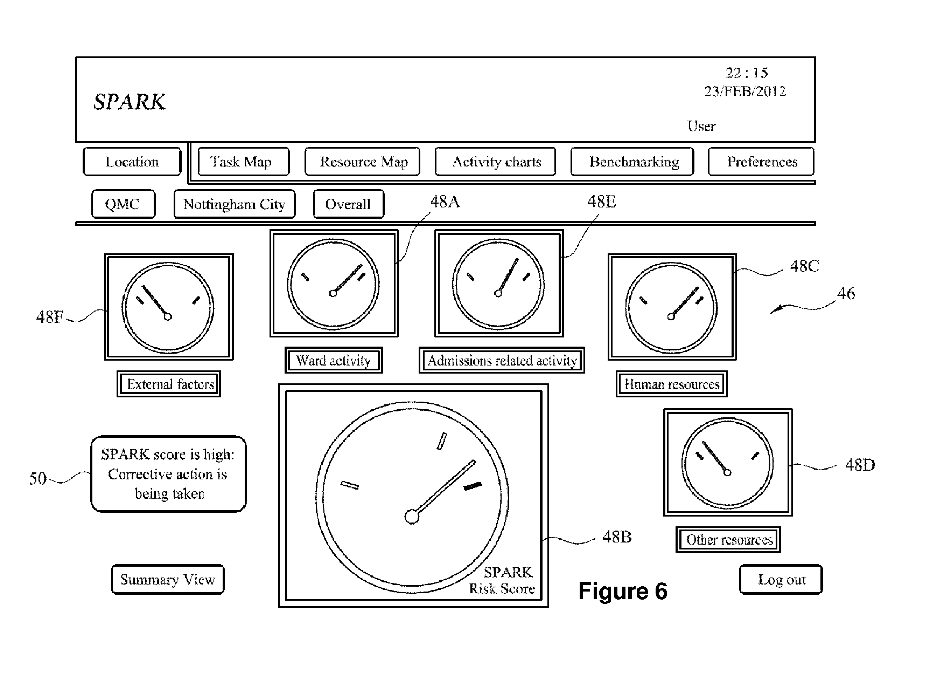

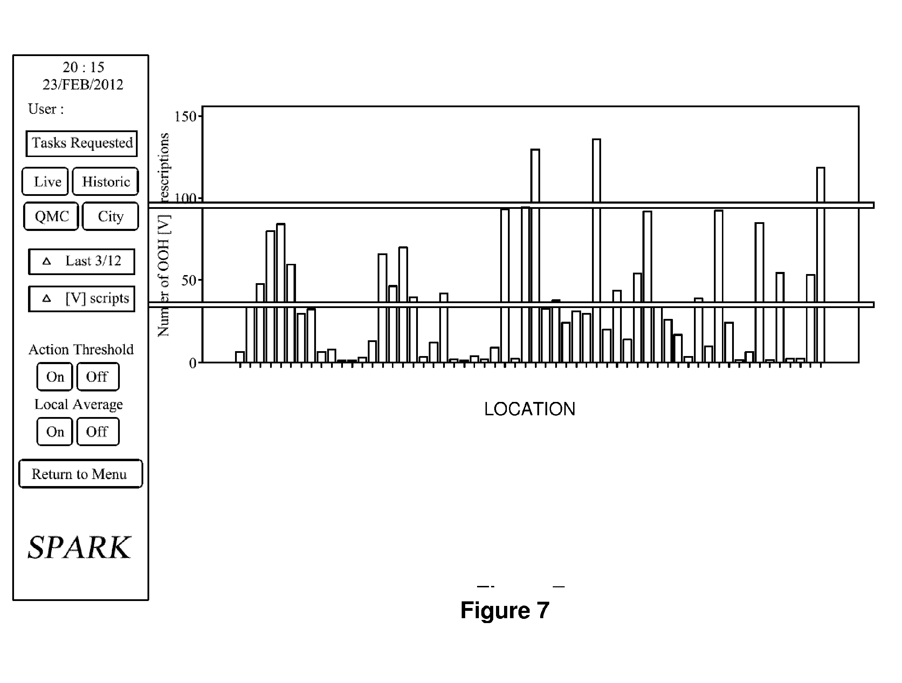

Turning back to Figure 2, there is shown the post analysis functions and/or outputs of the various different embodiments of the invention. As well as the alerts described above, various reporting functions are provided by the tool as shown in Figures 6 and 7, any of which user interfaces are typically selectable from within a common tool or application..

In Figure 6, there is shown a further GUI 46 for reporting the current activity levels in a facility or portion thereof, such as, in this example, a ward. In this display, a plurality of indicators 48 are provided on a single display to give an overview of the ward status. Each indicator displays the current level or value for a particular parameter determined by the tool. The indicators in this embodiment take the form of dials, on which markers may be provided for minimum, maximum, threshold values, etc may be provided. Whilst such dials are considered to be particularly intuitive to understand, it will be appreciated that other forms of indicator may be provided. Such alternative indicators preferably provide a graphical indication of current risk score relative to one or more threshold values, e.g. providing a graphical indication of risk percentage or ratio.

The total activity score for the ward is provided by indicator 48A and the total risk score for the ward is provided by indicator 48B. Separate indicators for the total activity scores for human resources 48C and equipment resources 48D respectively are also provided.

Further indicators may be provided for admissions related activity 48E (i.e. as an indicator of the numbers of new patient admissions or tasks associated therewith, which may be as yet unassigned) and/or external factors 48F. The external factors indicator 48F may be used to indicate factors outside of the control of the ward that may affect the potential level of activity or demand for resources, such as admissions or activity levels for other parts of the facility, the weather, the time of day, etc.

The total risk score indicated at 48B is calculated as a weighted summation of the activity score 48A and any, or any combination of, the other indicators 48C-48F. In one embodiment the total risk score 48B may be calculated as a percentage or fraction of threshold at which the likelihood of adverse event occurrence is known to be high. Accordingly the total risk indicator 48B may be considered to provide an adverse event risk indicator.

A summary or alert window 50 is provided to indicate the status and/or any required action to be taken in response thereto. This preferably comprises a textual display. In other embodiments, a report of the "grumble function" records received for that time period could also be displayed, for example by way of a counter.

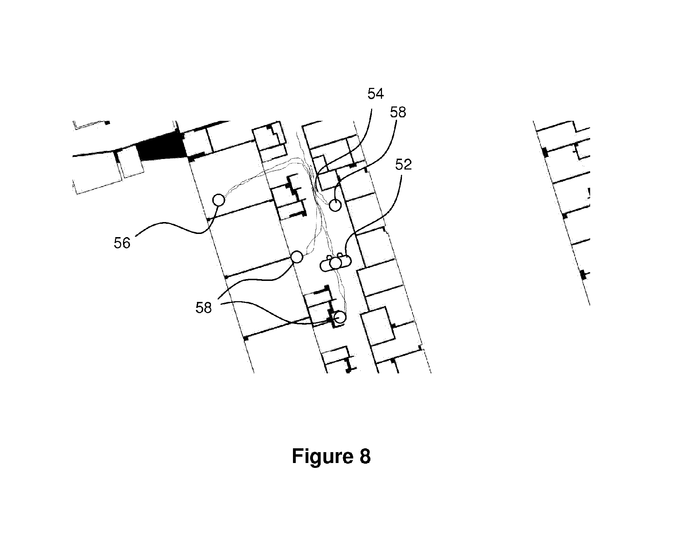

Any or any combination of the reporting or graphical output functions described above in relation to Figure 6, or below in relation to Figures 7 or 8, may provide an additional aspect of the invention.

In Figure 7, there is shown a reporting display in the form of a bar chart or histogram displaying the number of tasks undertaken by a plurality of different resources over a specified time period. Reports of this kind can be tailored by selecting only specific types of tasks and/or types of resources or indeed individual resources as required. The time period of interest may be specified accordingly and may be set as one or more shifts, days, weeks, months, years or other time increments. In this particular report the number of requests for Out Of Hours (OOH) prescriptions are recorded, which provides an indicator of incorrect initial drug prescriptions. Such information can thus be used to target root causes of problems.

In the present example, average and threshold values are also displayed as horizontal lines on the chart. It will be appreciated that various other types of conventional reporting or statistical charts can be produced using the amassed data as required. As well as actual number of tasks, or types of task, the tool may be used to display timing information, such the time to task completion. The tool may also determine average values and/or other statistical information, such as distributions of data, as well as variations thereof over time and/or comparisons between resources, parts of a facility, different facilities and/or corresponding results over different time periods.

The ability to output historical comparator data or comparator data from other sites, directorates, and wards is of particular benefit. The interface will allow managers and healthcare professionals to assess both specific and global stresses and stressors on the system.

Comparator data from multiple sites will typically be held at a central data repository. The SPARK^{™} tool will automatically transfer summary data to the repository with no patient identifiable parameters unless the users specify otherwise.

The graphical interfaces described above are made available on desktop PC, smartphone and/or tablet devices. The mobile version will allow the staff at work to be kept informed of activity elsewhere: this will be both by their own queries, and also by the system tailoring the information given to users.

At specified time points, for example monthly, the software will process stored data and generate reports to distribute to service leads and other interested parties. These reports will contain general data on the performance of the hospital, but also have sections tailored for each department, and for the role of the recipient (e.g. more information on drug errors for pharmacists).

Further, ad-hoc, reports will be generated each time the total activity or risk score exceeds its threshold value. This report will both initially inform users of the drivers of this increased risk and subsequently give supportive data to assess the efficacy of the targeted interventions instituted. The users of the system can decide to automatically send these reports to regulatory bodies to ensure transparency and external review of monitoring and action.

In view of the above discussion, it can be appreciated that a main function of the invention is to use the input data in parsimonious and accurate algorithms that gives a global assessment of the risk of an adverse outcome in the specified location. This provides a capability to use and augment secondary care data to derive both detailed information in relation to specific queries and meaningful summary statistics is potentially incredibly useful. It permits accurate assessment of daily activity, intra-and inter-hospital comparisons, and the dissemination of information to staff and users.

The outputs of the invention can increase efficiency in deploying resources in response to transient demands. Real-time monitoring and display of summary statistics informs managers of the stresses in their system, and crucially could assess the risk of clinical adverse events or errors prior to their occurrence: an "early warning score" for a ward or hospital. Moreover, these activity data can be used to monitor use of consumables (and automatically order replacements), to alert engineers to the failure of equipment, and to optimally redistribute physical resources across hospital sites. Harnessing and augmenting clinical data has the potential to lead to safer, more efficient, and more transparent performance throughout secondary care.

Turning to Figure 8, there is shown a graphical output of the tracking of a resource 52 within the model of the medical facility during attendance of one or more task. Here can be seen the path 54 taken by the resource (i.e. person or equipment) to the task locations 56 such that the distance travelled and the time spent can be accurately determined, for example with reference to fixed locations 58, e.g. such as a nurses station, notes/filing cabinet, equipment store or the like. Such modelling allows significant improvements in efficiency to be achieved by improving resource location and accessibility for the facility on either micro or macroscopic levels.

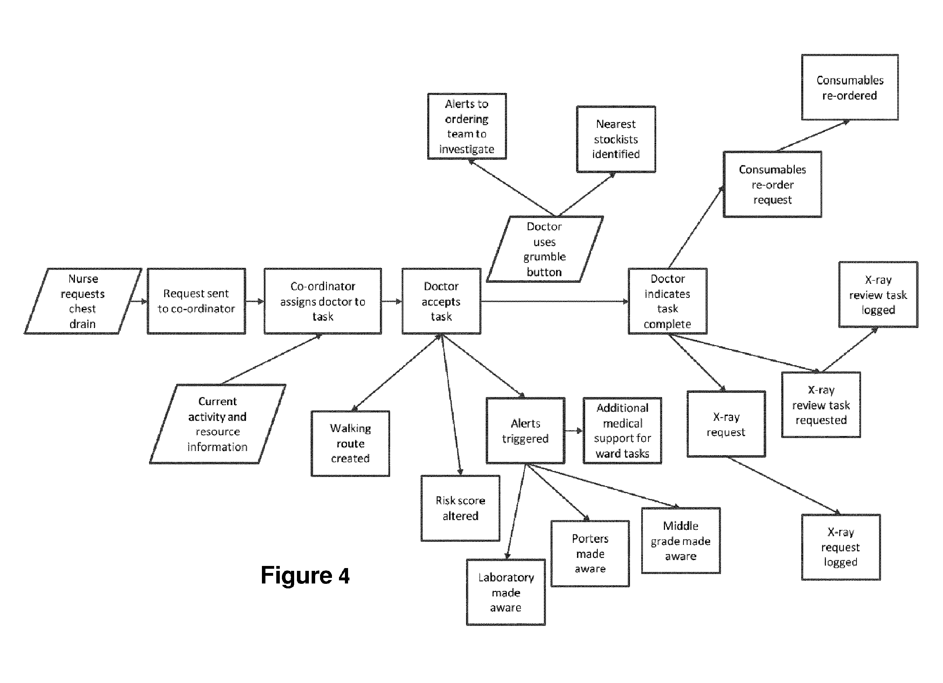

Below is described one hypothetical scenario of use of the system in a hospital, with reference to Figure 4, merely by way of example in order to demonstrate the advantages offered by the system.

Instructions are left by the consultant in respiratory medicine that a patient on his ward will require a chest drain on the following day if medical therapy has not improved his breathlessness due to a pleural effusion. The nurse finds the patient to be breathless on the following morning and logs a task request for a chest drain through a local task requesting program on her desktop computer. The SPARK tool checks the patient has had appropriate blood tests such as clotting, and, if not, prompts the requestor to take the samples or re-enter their password to confirm they accept the associated risk because of the urgency of the procedure.

The task is transferred to the out-of-hours coordinator who triages it to the most appropriate member of staff. To do this she can use information available from the graphical interface of SPARK, taking into consideration the data she can see on the number and type of tasks that are outstanding, the skill sets of the doctors on-call, and information on activity in other areas of the hospital.

The SPARK tool sends a number of alerts/task assignments in response to the request for the chest drain: to the laboratory technician to ensure the appropriate analyzer will be ready for the pleural fluid; to the porters to ensure someone will be available to take the samples to the laboratory; and to the middle grade doctor to ensure he or she is aware help may be necessary. An email is sent to the hospital account of the consultant in charge of the patient's care so they are aware of events when they return to the hospital on an ensuing day.

Any member of clinical staff looking at the SPARK graphical interface would see that the overall and specific risk assessment scores increase whilst that junior doctor cannot respond to any other request as he or she is undertaking this difficult sterile procedure. If the score is raised over the preset threshold, the system alerts the junior doctor on-call for orthopaedics, who is assessed, by virtue of his/her activity score, as not being too busy to undertake additional tasks in medicine to ensure the safe running of the hospital.

On arriving at the respiratory ward, the doctor finds he cannot undertake the procedure as there is no chest drain available. He uses the "grumble button" to register this inconvenience. The negative comments about chest drains are sent by email to the individual with responsibility for ordering such equipment for the ward so that restocking can occur at the earliest opportunity, and SPARK provides a list of the nearest wards that stock chest drain equipment to the doctor's smartphone, including a route to the nearest ward.

SPARK automatically adds the consumables used in the procedure, such as a silk suture and sterile gown, to the hospital's ordering system.

The doctor uses their smartphone to indicate they have completed the procedure via the SPARK GUI, and the SPARK tool uses the clinical and patient details on the chest drain request to make an automated request for a chest X-ray (i.e. as a linked downstream task to be fulfilled). A further task is generated on completion of the X-ray for a junior doctor to review it to ensure the drain is appropriately sited.

Thus the tool can accommodate the assignment and/or reallocation of tasks in response to the varying circumstances encountered in a medical facility.

Based on the above discussion, it can be understood that the invention offers two graphical outputs that allow significant improvements in the efficiency with which tasks can be undertaken using available resources. One output comprises a graphical model of the facility on which task and resource locations can be displayed, for example to ensure that tasks and resources can be assigned at least in part based upon relative locations in the model. The other output comprises simple graphical indicators of the summation of task execution parameters and/or resource parameters for the facility, for example as dials, charts, or similar. The output of such parameter displays against thresholds or limits for said parameters can provide a real-time reporting and/or control system that has not been hitherto possible. An aspect of the invention may be considered to comprise any, or any combination, of said graphical outputs.

## Claims

1. A medical service deployment system for use in a medical facility, the system comprising:
a data storage device having stored thereon a model of the medical facility, a record of the availability of one or more resources within the medical facility and records of a plurality of tasks to be undertaken within the medical facility, the records comprising a task execution parameter and a location of the task to be undertaken in the medical facility;
a plurality communication devices in communication with the data storage device, said communication devices each being associated with a resource and having location determining means; and
one or more processors arranged to compare the availability of the one or more resources with the task execution parameter of one or more tasks to be undertaken and to assign a resource to the one or more tasks in dependence thereon.

2. The system of claim 1, wherein the processor controls the transmission of an instruction to at least one of the communication devices, the instruction being indicative of the assignment of one or more task to the resource associated with said communication device.

3. The system of claim 1 or 2, wherein the location of the one or more resource within the facility is recorded or tracked by the one or more processors via a wireless communication signal to or from the associated communication device, wherein said location is recorded within the model of the facility.

4. The system of any preceding claim, wherein the location of a task to be undertaken is compared with the location of the one or more resources and a task is assigned to a resource based, at least in part, upon the distance there-between.

5. The system of any preceding claim, wherein the record of the availability of one or more resources comprises a total of the task execution parameters for the tasks currently assigned to the one or more resources.

6. The system of claim 5, wherein the record of availability for each resource has associated therewith a threshold value and the one or more processors compares the total of the task execution parameters assigned to one or more resources with the threshold value in order to determine the availability of said one or more resources, wherein the processor assigns a task to a resource, at least in part, based on the difference between the current total of task execution parameters and the threshold value for the resource.

7. The system of any preceding claim, wherein the resource comprises any, or any combination, of medical machines, devices, rooms and/or consumable materials.

8. The system of any preceding claim, wherein a profile is stored for each resource, the profile comprising a list of tasks or types of task that can be undertaken by a resource.

9. The system of any preceding claim, wherein the task execution parameter for each task comprises a score indicative of the urgency or duration of the task.

10. The system of any preceding claim, wherein the model comprises a graphical plan of the medical facility, the location of the task to be undertaken corresponding to a location or portion of the plan.

11. The system of any preceding claim, wherein the model comprises a plurality of portions, each portion being distinguished from a further portion by its location and/or function, and wherein a summation of the task execution parameters for active tasks assigned to the resources within said portion is maintained.

12. The system of claim 11, wherein the processor compares the total for one portion of the facility with the total for one or more further portions of the facility and assigns tasks to resources within one or more of said portions based upon said comparison and wherein a risk indication for each portion of the facility is determined based on a ratio of the summation the task execution parameters for the resources within that portion against a summation of threshold values for each resource.

13. The system of any preceding claim, wherein a visual representation of the facility model is output on a display, said visual representation comprising an indication of the location of one or more tasks and/or resources.

14. The system of claim 13, wherein the visual representation comprises a proposed route from a resource to a task or vice versa.

15. The system of any preceding claim, further comprising one or more movement sensor associated with each resource, wherein the output of the movement sensor is used to determine a location of each resource within the facility.
